(19) Europäisches Patentamt
European Patent Office
Office européen des brevets

(11) EP 4 455 157 A1

(12) EUROPEAN PATENT APPLICATION

(43) Date of publication:
**30.10.2024 Bulletin 2024/44**

(21) Application number: **23382393.9**

(22) Date of filing: **27.04.2023**

(51) International Patent Classification (IPC):
***C07K 14/47** (2006.01)* ***A61K 39/00** (2006.01)*
***C07K 14/725** (2006.01)* ***C12N 15/86** (2006.01)*

(52) Cooperative Patent Classification (CPC):
**C07K 14/47; C07K 14/7051; C12N 15/86;**
C12N 2740/16043; C12N 2830/008; C12N 2830/15

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **FUNDACION PUBLICA ANDALUZA PROGRESO Y SALUD M.P.- FPS**
  **41092 Sevilla (ES)**
- **Lentistem Biotech S.L.**
  **18016 Granada (ES)**
- **Universidad de Granada**
  **18071 Granada (ES)**

(72) Inventors:
- **MARTÍN MOLINA, Francisco**
  **18071 Granada (ES)**
- **MALDONADO PÉREZ, Noelia**
  **41092 Sevilla (ES)**
- **TRISTÁN MANZANO, María**
  **18016 Granada (ES)**
- **JUSTICIA LIRIO, Pedro Luis**
  **18016 Granada (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

Remarks:
The complete document including Reference Table(s) and the Sequence Listing(s) can be downloaded from the EPO website

# (54) POLYNUCEOTIDE FOR T CELL SPECIFIC TRANSGENE EXPRESSION

(57) The present invention refers to the medical field. Particularly, the present invention refers to a polynucleotide for T cell specific transgene expression, as well as to a method to generate CAR-T cells.

EP 4 455 157 A1

## Description

### FIELD OF THE INVENTION

The present invention refers to the medical field. Particularly, the present invention refers to a polynucleotide for T cell specific transgene expression, as well as to a method to generate CAR-T cells by using said polynucleotide.

### STATE OF THE ART

T lymphocytes constitute the basis of multiple cellular therapies due to their easy isolation and manipulation and their functional and migration properties, which make them excellent candidates for immunotherapy. With the new advanced technologies, genetic engineering of T lymphocytes can be easily addressed opening an endless of possibilities of therapeutic applications. Genetically engineered T cell immunotherapies have provided impressive efficacy treating B cell malignancies, endowing T cells with a chimeric antigen receptor to specifically recognize the antigen. These approaches can be applied to multiple diseases, from other cancers to infectious and autoimmune problems.

Chimeric antigen receptor (CAR) T cells (CAR-T cells) have become one of the most promising approaches for the treatment of cancer. CAR-T cell therapy represents a major advancement in personalized cancer treatment. In this strategy, patient's own T cells are genetically engineered to express a synthetic receptor that binds a tumor antigen. CAR T cells are then expanded for clinical use and infused back into the patient's body to attack and destroy chemotherapy-resistant cancer. Traditionally, the CAR is transduced into T cells via viral vectors, such as lentiviruses (LVs). Lentiviral particles are packaged in producer cell lines such as HEK293T cells. Upon co-transfection of different plasmids, one of which contains the CAR transgene, all required sequences are available to produce and package a viral particle containing the transgene of interest. Some lentiviral vectors, such as those derived from HIV-1 have, already been approved by the FDA/EMA.

Dramatic clinical responses and high rates of complete remission have been observed in the setting of CAR T-cell therapy of B-cell malignancies. This resulted in recent FDA/EMA approvals of CAR T cells directed against the CD19 protein ($\alpha$CD19-CAR-T cells) for treatment of acute lymphoblastic leukemia and diffuse large B-cell lymphoma. These advanced therapy medicinal products (ATMPs) include: tisagenlecleucel (Kymriah, Novartis), axicabtageneciloleucel (Yescarta, Kite-Gilead), brexucabtagene autoleucel (Tecartus, Kite-Gilead) and lisocabtagenemaraleucel (Breyanzi, Bristol Myers Squibb). Recently idecabtagene vicleucel (Abecma, Bristol Myers Squibb) and ciltacabtagene autoleucel (Carvykti, Janssen) were also approved as $\alpha$BCMA-CAR-T ATMPs for the treatment of refractory Multiple Myeloma. However, despite the impressive results of $\alpha$CD19-CAR-T cells, there are still several aspects that must be improved. Aggressive severe side effects due to CAR-T overstimulation, such as cytokine release syndrome and neuroinflammation are common among treated patients and can lead to fatal outcomes. Furthermore, sustained complete remissions range from 42% to 62% for patients treated with commercial ATMPs, evidencing much room for improvement.

Different works have uncovered the importance of controlling CAR expression levels at the surface of the CAR-T cells to optimize its therapeutic activity. Despite this, all four approved $\alpha$CD19-CAR-T cells and most of those that are being tested in ongoing clinical trials use autologous T cells transduced with retroviral vectors expressing a $\alpha$CD19-CAR through constitutive, strong promoters such as the human EF1$\alpha$ and the murine stem cell virus LTR (MSCV). High CARs concentrations on the T cell surface can result in spontaneous clustering of the CARs (independent of the ligand) leading to tonic-signalling. This tonic signalling can affect safety (pro-inflammatory cytokines secretion in non-target tissues) and efficacy (premature exhaustion due to continuous proliferation) of CAR-T cells. Furthermore, high-density and constitutive CAR presence on the T cell surface can also lead to overstimulation upon antigen recognition, which also affects safety (excess of pro-inflammatory cytokine secretion) and efficacy (early exhaustion, apoptosis and loss of naive/stem cell memory T cells phenotype) of the CAR-T cells.

So, there is an unmet medical need to improve the efficacy and safety of CAR-T cells or fourth generation CAR-T cells or TRUCKs (T cells redirected for antigen-unrestricted cytokine-initiated killing') . In particular, there is an unmet medical need for the development of strategies aimed at generating CAR-T cells that express the CAR in a more physiological manner than that provided by strong promoters. Furthermore, tissue-restricted gene expression can be useful in improving the manufacturing of CAR-T or TRUCK cells, as it would allow for the production of LVs without expressing the transgene at the packaging cells which, in case of toxicity, can significantly reduce viral titer. The present invention is focused on solving this problem, and a polynucleotide sequence for T cell specific transgene expression is herein provided.

## DESCRIPTION OF THE INVENTION

### Brief description of the invention

The present invention refers to a polynucleotide sequence for T cell specific transgene expression, as well as to a method to generate CAR-T cells by using said polynucleotide.

The inventors of the present invention identified the necessity to generate CAR-T cells that express the CAR in a more physiological manner than that provided by strong promoters. They hypothesized that restricting transgene expression to T cells, using a T-cell specific promoter, could solve some of the safety concerns associated to CAR-T cell therapies. For this purpose, the inventors generated a synthetic polynucleotide comprising or consisting **of SEQ ID NO: 7** that is based on regulatory regions of the human CD4 locus **(Figure 1).** As shown in **Figure 1, SEQ ID NO: 7** comprises or consists of the sequences: **SEQ ID NO: 1, SEQ ID NO: 2, SEQ ID NO: 3, SEQ ID NO: 4, SEQ ID NO: 5** and **SEQ ID NO: 6. SEQ ID NO: 8** comprises or consists of **SEQ ID NO:7** and accessory sequences containing restriction sites.

The sequences included in the present application are summarised below:

**SEQ ID NO: 1:**

TTAGGGTTCAGGCCTGAGCCCCTGCTGCTATCCCTCCTTCAAAGGAGGAGATCAA
GGAGCTTAGGGTCCCCCGCACAGGCCCACCCCAGGGTGGGGTTCTTCCTTTGAAG
GGAATTGCTTTGGGGTGGGGTCGGTTCTATCTGCTCCACTCTGTGGCTGACAGTTT
CTCCAAGGGGCTGCAGGTGTCAGCTGTCTGAGCCCGGGCTGAGCTCTGAAACGT
GCCTACTCAAACTTCCCGTGGGGTAGGGGAGGCCCAGAACCACCCTCTGAGAGT
GGCAAAAAGTGGTCCTGGAGCCAGGGGAAATGTGGATGGGGTAGAGAT

**SEQ ID NO: 2:**

CTTATCAAAATTGAGTCACTTGGACACATCCAGCTGTGAGGGAGGCCTCAACTTC
CTGGAAGTTGGGTTTTATTTGCTTGGGCAAGTTGGCCATATGCCCCCAACTGCC
TATTTA

**SEQ ID NO: 3:**

AGCCTGATTCTGCTTAACTTTTTCCCTTGACTTTGGCATTTTCACTTTGACATGTTC
CCTGAGAGCCTGGGGGGTGGGGAACCCAGCTCCAGCTGGTGACGTTTGGGGCCG
GCCCAGGCCTAGGGTGTGGAGGAGCCTTGCCATCGGGCTTCCTGTCTCTCTTCAT
TTAAGCACGACTCTGCAGAAGGAA

**SEQ ID NO: 4:**

CAAAGCACCCTCCCCACTGGGCTCCTGGTTGCAGAGCTCCAAGTCCTCACACAGA
TACGCCTGTTTGAGAAGCAGCGGG

**SEQ ID NO: 5:**

CAAAGACGCAAGCCCAGAGGTAAGGTGGTCAGACTCGGCTTCCTTCCCCGGAGC TGAGAGGGAGGGGAACGTGGGGCAGATGCACAGGAAGAATTTCTGTGCTCTGCC CAGTTGTCTGCTTTAGATAAATATGTC

**SEQ ID NO: 6:**

TGGGGGTTATGGGGAGAAGATAAAAGTGCCTGTGGGACCACAGACTCTCGCTGT GGTGGAGCTGGGCCCTCTTACCCTCCCAAGCCTCGCCCCTCATCCCATCCCTGGG GGCCAGGGGTGAGGGCGGCAGGAACCTCAAGGCTCTGAGAAAGTGCGTGGTGTG TGTTGCCATTTTGGTCTCTTCTCC

**SEQ ID NO: 7:**

TTAGGGTTCAGGCCTGAGCCCCTGCTGCTATCCCTCCTTCAAAGGAGGAGATCAA GGAGCTTAGGGTCCCCCGCACAGGCCCACCCCAGGGTGGGGTTCTTCCTTTGAAG GGAATTGCTTTGGGGTGGGGTCGGTTCTATCTGCTCCACTCTGTGGCTGACAGTTT CTCCAAGGGGCTGCAGGTGTCAGCTGTCTGAGCCCGGGCTGAGCTCTGAAACGT GCCTACTCAAACTTCCCGTGGGGTAGGGGAGGCCCAGAACCACCCTCTGAGAGT GGCAAAAAGTGGTCCTGGAGCCAGGGGAAATGTGGATGGGGTAGAGATCTTATC AAAATTGAGTCACTTGGACACATCCAGCTGTGAGGGAGGCCTCAACTTCCTGGA AGTTGGGTTTTATTTTGCTTGGGCAAGTTGGCCATATGCCCCCAACTGCCTATTTA AGCCTGATTCTGCTTAACTTTTTCCCTTGACTTTGGCATTTTCACTTTGACATGTTC CCTGAGAGCCTGGGGGGTGGGGAACCCAGCTCCAGCTGGTGACGTTTGGGGCCG GCCCAGGCCTAGGGTGTGGAGGAGCCTTGCCATCGGGCTTCCTGTCTCTCTTCAT TTAAGCACGACTCTGCAGAAGGAACAAAGCACCCTCCCCACTGGGCTCCTGGTT GCAGAGCTCCAAGTCCTCACACAGATACGCCTGTTTGAGAAGCAGCGGGCAAAG ACGCAAGCCCAGAGGTAAGGTGGTCAGACTCGGCTTCCTTCCCCGGAGCTGAGA GGGAGGGGAACGTGGGGCAGATGCACAGGAAGAATTTCTGTGCTCTGCCCAGTT GTCTGCTTTAGATAAATATGTCTGGGGGTTATGGGGAGAAGATAAAAGTGCCTGT GGGACCACAGACTCTCGCTGTGGTGGAGCTGGGCCCTCTTACCCTCCCAAGCCTC GCCCCTCATCCCATCCCTGGGGGCCAGGGGTGAGGGCGGCAGGAACCTCAAGGC TCTGAGAAAGTGCGTGGTGTGTGTTGCCATTTTGGTCTCTTCTCC

**SEQ ID NO: 8:**

TTGAATTCGGCATGCTTATCGATTTTAGGGTTCAGGCCTGAGCCCCTGCTGCTATC
CCTCCTTCAAAGGAGGAGATCAAGGAGCTTAGGGTCCCCGCACAGGCCCACCC
CAGGGTGGGGTTCTTCCTTTGAAGGGAATTGCTTTGGGGTGGGGTCGGTTCTATC
TGCTCCACTCTGTGGCTGACAGTTTCTCCAAGGGGCTGCAGGTGTCAGCTGTCTG
AGCCCGGGCTGAGCTCTGAAACGTGCCTACTCAAACTTCCCGTGGGGTAGGGGA
GGCCCAGAACCACCCTCTGAGAGTGGCAAAAAGTGGTCCTGGAGCCAGGGGAAA
TGTGGATGGGGTAGAGATCTTATCAAAATTGAGTCACTTGGACACATCCAGCTGT
GAGGGAGGCCTCAACTTCCTGGAAGTTGGGTTTTATTTTGCTTGGGCAAGTTGGC
CATATGCCCCCAACTGCCTATTTAAGCCTGATTCTGCTTAACTTTTTCCCTTGACT
TTGGCATTTTCACTTTGACATGTTCCCTGAGAGCCTGGGGGGTGGGGAACCCAGC
TCCAGCTGGTGACGTTTGGGGCCGGCCCAGGCCTAGGGTGTGGAGGAGCCTTGC
CATCGGGCTTCCTGTCTCTCTTCATTTAAGCACGACTCTGCAGAAGGAACAAAGC
ACCCTCCCCACTGGGCTCCTGGTTGCAGAGCTCCAAGTCCTCACACAGATACGCC
TGTTTGAGAAGCAGCGGGCAAAGACGCAAGCCCAGAGGTAAGGTGGTCAGACTC
GGCTTCCTTCCCCGGAGCTGAGAGGGAGGGGAACGTGGGGCAGATGCACAGGAA
GAATTTCTGTGCTCTGCCCAGTTGTCTGCTTTAGATAAATATGTCTGGGGGTTATG
GGGAGAAGATAAAAGTGCCTGTGGGACCACAGACTCTCGCTGTGGTGGAGCTGG
GCCCTCTTACCCTCCCAAGCCTCGCCCCTCATCCCATCCCTGGGGGCCAGGGGTG
AGGGCGGCAGGAACCTCAAGGCTCTGAGAAAGTGCGTGGTGTGTGTTGCCATTT
TGGTCTCTTCTCCGGCGCGCCCGGGATCCACGCGTCTGAATTCGA

To assess the functional role of the synthetic polynucleotide, the inventors cloned it into the CEWP lentiviral plasmid, upstream of an eGFP domain **(Figure 2A).** The LV produced using this lentiviral plasmid will be referred to as the CD4-GFP LV. Next, the inventors used the CD4-GFP LV to transduce multiple cell lines and primary T cells. High eGFP expression was only detected in cells that naturally express CD4, indicating that the synthetic promoter was specific to CD4+ cells **(Figure 2B, 2C).** In addition, percentage of eGFP+ cells was comparable in transduced primary CD4+ and CD8+ T cells, even the intensity of expression was significantly higher in CD4+ T cells **(Figure 2D).**

To investigate whether the synthetic polynucleotide mimics CD4 expression, the inventors compared the expression of the exogenous CD4-GFP with the expression of the endogenous CD4 in transduced primary T cells. To stimulate CD4-promoter mediated transcription, T cells were activated with αCD3/αCD28. Upon activation, CD4-GFP expression - regulated under the control of the synthetic promoter - mimicked the pattern of endogenous CD4 expression **(Figure 3C).** Although the expression of GFP did not completely mimic the expression pattern of CD3, GFP expression did not increase after antigenic stimulation of T cells, which usually occurs with strong viral or eukaryotic promoters **(Figure 3B, 4).**

Overall, these results indicate that the synthetic polynucleotide can be used for T cell specific transgene expression, and that it mimics the pattern of expression of the endogenous human CD4 after activation.

So, the **first embodiment** of the present invention refers to a polynucleotide comprising **SEQ ID NO: 7** or having an identity of sequence with **SEQ ID NO: 7** of at least 95%.

In a preferred embodiment, the polynucleotide comprises a promoter sequence consisting of **SEQ ID NO: 7** or having an identity of sequence with **SEQ ID NO: 7** of at least 95%.

In a more preferred embodiment, the polynucleotide comprises a promoter sequence consisting of **SEQ ID NO: 7.**

In a more preferred embodiment, the polynucleotide consists of **SEQ ID NO: 7.**

In a more preferred embodiment, the polynucleotide of the invention comprises or consists of **SEQ ID NO: 7** and accessory sequences containing restriction sites, for instance **SEQ ID NO: 8.**

The **second embodiment** of the invention refers to a genetic construct comprising any of the polynucleotides described above.

In a preferred embodiment, the genetic construct comprises the any of the polynucleotides described above as promoter operatively linked to a nucleotide sequence encoding a protein to drive its expression.

In a more preferred embodiment, the protein is a chimeric antigen receptor (CAR).

The **third embodiment** of the invention refers to a lentiviral vector comprising any of the polynucleotides, or the genetic constructs described above.

The **fourth embodiment** of the invention refers to a T cell or T cell-derived cell comprising any of the polynucleotides, or genetic constructs described above, or any genetic material introduced using the lentiviral vector described above.

The **fifth embodiment** of the invention refers to a any of the polynucleotides, genetic constructs, lentiviral vector, T cell or T cell-derived cell described above for use in therapy, for instance in cancer therapy.

So, a preferred embodiment involves the polynucleotides, genetic construct, lentiviral vectors, T cell or T cell-derived cell for use in cancer therapy.

Another preferred embodiment involves the polynucleotides, genetic construct, lentiviral vectors, T cell or T cell-derived cell for use in human cancer therapy.

The **sixth embodiment** of the invention refers to the use of any of the polynucleotides described above as a promoter to drive gene expression in T cells or T cell-derived cells.

In a preferred embodiment, the polynucleotide drives the expression of a nucleotide sequence encoding a CAR.

The **seventh embodiment** of the invention refers to the use of any of the genetic constructs or lentiviral vector described above to express a nucleotide sequence encoding a CAR in T cells or T cell-derived cells.

The **eighth embodiment** of the invention refers to a method for generating cells that express a transgene in a manner that mimics the expression of the human CD4 gene, which comprises transducing cells with a genetic construct comprising any of the polynucleotides described above as promoter operatively linked to a nucleotide sequence encoding a protein, or with any of the genetic constructs or the lentiviral vector described above.

In a preferred embodiment, the method comprises transducing T-cells or T-cell derived cells with any of the genetic constructs or lentiviral vector described above.

A preferred embodiment involves a method for generating CAR T-cells which comprises transducing T-cells with a genetic construct comprising any of the polynucleotides described above as promoter operatively linked to a nucleotide sequence encoding a CAR, or with any of the genetic constructs or the lentiviral vector described above.

In a preferred embodiment, the cells of the above-mentioned embodiments are human cells.

The present invention also refers to a method for treating patients, for instance patients suffering from cancer, which comprises the administration of CAR T-cells generated using any of the methods described above.

The synthetic polynucleotide described above can be used to restrict the expression of the CAR transgene exclusively to T cells. This can improve the efficacy of CAR-T cell therapies by:

1. Blocking the expression of the CAR transgene in packaging cells during LV generation, thereby resulting in the generation of lentiviral particles that will be free of CARs.
2. If the T cells collected from the patient are contaminated with other cell types, by blocking the expression of the CAR transgene in case of LV-mediated transduction of contaminating cells.

The membrane of the retroviral vectors is derived from the packaging cells and contain different proteins derived from those producing cells. The level of incorporation is highly variable and depends on the type of retroviral vector, the protein and its concentration. In this direction, it has been described that CAR-expressing retroviral particles present the CAR on its surface, thereby facilitating its binding to the cognate antigen. In fact, malignant B cells are transduced at higher levels with LVs displaying anti-CD19 CARs compared to LVs displaying non-binding control constructs. This is referred to as transduction targeting.

Several groups are investigating the feasibility of direct inoculation of CAR-coding vectors into the patient as a cheaper and quicker alternative to *ex vivo* generation of CAR-T cells and posterior re-infusion. However, there are multiple safety concerns to solve before this strategy can be applied into patients. The present invention could accelerate this translation by providing a tight control of CAR expression only on T cells. This can also be combined with transduction targeting to avoid transduction of leukemic cells. Therefore, this invention would be a valuable alternative or complement to the receptor-targeted LVs (RT-LVs).

In addition to the described application to improved CAR-T cells platforms, other potential applications of the invention relate to any gene therapy strategies aiming to modify T cells *ex vivo* or *in vivo*. For example, new gene therapy strategies for immunodeficiencies and autoimmune diseases could be developed based on the specific expression of the desired gene only on T cells.

The polynucleotide can be used to achieve T-cell specific expression during pluripotent stem cell differentiation to investigate T cell development *in vitro* or *in vivo.*

The invention can be also of interest for production of retroviral vectors expressing highly toxic proteins, by blocking production its production on the packaging cells. This would improve the quality of particle production since the particles will be free of toxic proteins.

In the context of the present invention the following terms are defined:

- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.

## Description of the figures

**Figure 1. DNA sequence of CD4 promoter.** A) Scheme of the full length synthetic CD4 promoter showing the positions of the different regions included, each one identified with a different color. **B)** Nucleotide sequence of the different regions of the synthetic CD4 promoter. **C)** Table indicating the sequences included in the synthetic CD4 promoter showing the sequence ID and exact locations in the GeneBank nucleotide sequence database. The chimeric promoter contains 6 sequences (from Seq_1 to Seq_6) obtained from different positions of the CD4 genomic locus. A human CD4 HS4 enhancer defined in (1) and (2) was added to the construct in the 5' region, with the 3' region more homologous to the murine enhancer. Adjacent-to-promoter sequences defined by EMBL nucleotide sequence database were also added. A CD4 minimal promoter defined in (3) and (4) was added, as well as a region of CD4 defined as promoter by Eukaryotic Promoter Database (EPD) (chr12:6789491-6789550), which varies in the 5' region with respect to the promoter region defined in (3). An enhancer region defined in (5) was incorporated into the 3' region.

**Figure 2. GFP expression levels under the control of the CD4 synthetic promoter in 4 different cell types. A)** Representation of lentiviral plasmids used to generate LVs. The CD4-GFP plasmid has an eGFP domain under the control of the synthetic CD4 promoter. The CEWP plasmid, used as control, has an eGFP domain under the control of the CMV promoter. **B)** CD4+++ cells: Primary T cells and Jurkat (an immortalized T cell line), CD4+: Namalwa, and non-T cells (293T and BxPC-3) were transduced with CD4-GFP or CEWP LVs and 5 days later analysed for eGFP expression. The different histograms show the expression levels of eGFP on the different cell lines transduced with each plasmid. NTD: non-transduced cells. **C)** Percentage (left) and intensity (right) of positive GFP+ cells in the different cell types analysed. Two-tailed T test. **D)** Percentage (left) and intensity (right) of eGFP+ cells among the CD4+ and CD8+ T cell subsets.

**Figure 3. Kinetics of CD4-driven polynucleotide in primary T cells.** A) Work-flow of the kinetics experiments. Briefly, cells were activated, transduced with the CD4-GFP LVs and cultured for 10 days until resting. Then, cells were stimulated with TransAct and eGFP/CD3 expression was determined by FACS at the indicated times. **B)** Fold change between the percentage of positive cells (CD3+ or eGFP+) at different timepoints post stimulation, and those at 0h. ANOVA-two tails test, Bonferroni post T. **, $p<0.01$; ***, $p<0.001$. **C)** Fold change between the expression of endogenous CD4 mRNA and exogenous CD4-GFP mRNA at different timepoints post stimulation, and those at 0h.

**Figure 4. GFP kinetics in CD4/CD8 and CD45RA/CD62L T cells subsets. A)** Fold change of GFP positive cells analysed in the CD4+ (left) and CD8+ (right) T cells subsets compared to the fold change CD3, both compared to 0h. **B)** Fold change of GFP positive cells analysed in the CD45RA+CD62L+ (top left), CD45RA-CD62L+ (top right), CD45RA-CD62L- (bottom left) and CD45RA+CD62L- (bottom right) T cells subsets compared to the fold change CD3, both compared to 0h. ANOVA-two tails test, Bonferroni post T. *, $p<0.05$, **, $p<0.01$; ***, $p<0.001$.

## Detailed description of the invention

The present invention is illustrated by means of the Examples set below without the intention of limiting its scope of protection.

## Example 1. Material and Methods

### Example 1.1. Polynucleotide design and synthesis

The synthetic polynucleotide **(SEQ NO: 7,** also referred to as the synthetic CD4 promoter) was designed based on CD4 endogenous locus. The inventors used different regions of the CD4 locus defined as important for regulation by different databases, genomic browsers or described as such in the literature. As shown in **Figure 1, SEQ ID NO:7** is composed of **SEQ ID NO: 1, SEQ ID NO: 2, SEQ ID NO: 3, SEQ ID NO: 4, SEQ ID NO: 5** and **SEQ ID NO: 6. SEQ ID NO: 8** consists of **SEQ ID NO:7** and accessory sequences containing digestive restriction sites.

The polynucleotide was synthetized by GenScript (Genescript USA Inc. NY, USA) in a pUC57 vector, flanked by EcoRI/ClaI/SphI and BamHI/AscI/EcoRI resctriction sites.

### Example 1.2. Lentiviral plasmid constructions

The synthetic polynucleotide was cloned into CEWP LV vector by using ClaI and BamHI with and standard techniques of molecular biology to generate what will be referred to as the CD4-GFP lentiviral plasmid. As shown in **Figure 2A,** the CEWP lentiviral plasmid contains an eGFP domain, meaning that the resultant CD4-GFP lentiviral plasmid contains eGFP domain under the regulation of the synthetic CD4 promoter.

### Example 1.3. Cell culture

HEK-293T (ATCC® CRL-11268) were cultured in DMEM (Biowest) supplemented with 10% FBS (Biowest) and maintained at 10% $CO_2$ and 37°C. Namalwa (ATCC® CRL-1432), Jurkat (ATCC® TIB-152), BxPC3 (ATCC® CRL-1687) cells were cultured in RPMI and MiaPaCa2 (ATCC® CRL-1420) in DMEM supplemented with 10% FBS and 1% P/S (Biowest) and maintained at 5% CO2 37°C. Primary T cells were isolated from the peripheral blood mononuclear cell (PBMC) section after Ficoll gradient (Lymphosep, Biowest) from peripheral blood and negative selected with the MACSexpresss Pan T cell isolation (Miltenyi Biotec). Isolated T cells were cultured in TexMACS medium (Miltenyi Biotec) supplemented with 20 UI/ml of IL-2 (Miltenyi) and 1% of P/S and maintained at 37°C in a 5% $CO_2$ incubator. T cells were activated with TransAct (1:100, Miltenyi Biotec) during 48h before lentiviral transduction following manufacturer's recommendations.

### Example 1.4. Generation of LVs

Using 6-well plates, 0.7-0.8$\times 10^6$ HEK293T (90% of confluency) were transfected with the lentiviral plasmid of interest, pCMV_R8.91 and pMD2.G in proportion 3:2:1 using LipoD293 (SigmaGen Lab). DNA and LipoD were mixed in DMEM without serum and incubated for 10 min at room temperature before being added drop-by-drop to the cells. Media was exchanged 5h post-transfection and supernatants were harvested at 48h and 72h after transfection and filtered through a 0.45$\mu$m filter (Nalgene, Rochester, NY) and concentrated 30x using Amicon Ultra15 (100 KD, Milipore) filters and centrifugation at 1800g 4°C. LVs were aliquoted and stored at -80°C.

Briefly, for the estimation of the efficient viral titer, $10^5$ cells were incubated with different volume of viral supernatant. Percentage of eGFP+ cells was determined by flow cytometry 72h post-transduction and transducing units per ml (TU/ml) were estimated according to the formula:

$$Titer = \frac{100000\ plated\ cells \times \%GFP + cells\ \times 1000}{microliters\ of\ LVs\ used}$$

The LVs produced upon transfection with the CD4-GFP and the CEWP lentiviral plasmids will be referred to as the CD4-GFP LV and the CEWP LV, respectively.

### Example 1.5. Transduction

Briefly, $10^5$ cells of suspension Jurkat and Namalwa cells were incubated with different volume of viral supernatants during 4.5h post-spinoculation (30min at 32°C 800g). In the case of adherent cells, $5\times 10^4$ cells of MiaPaCa2 and BxPC3 cells or $1\times 10^1$ HEK-293T cells were plated with viral supernatants in a 24-well-plate and media was exchange after 5h of incubation with LVs. For T cell transduction, $2\times 10^5$ activated T cells were incubated with LVs in s 96 well-plate and spinoculated at 800g for 1h at 32°C. After 5h, cells were washed and plated at a density of $10^6$ c/ml. Cell transduction was evaluated by flow cytometry.

**Example 1.6. Analysis of expression kinetics in primary T cells**

For T cells transduced with eGFP-LVs, $10^5$ T cells were stimulated with TransAct (Miltenyi Biotec) after 10 days of the initial activation for transduction. Fold change of eGFP expression was indicated as the ratio of percentage of positive populationrelated to percentage of positive population at 0h.

**Example 1.7. Statistical analysis**

Data are represented as mean ±SEM. Two-tailed ANOVA, Bonferroni post-test has been used to compared kinetics as indicated in every figure caption.

**Example 2. Results**

**Example 2.1. Construction of T-specific chimeric promoters based on different regulatory regions of the CD4 locus.**

To construct a T-cell specific promoter, the inventors used different regions of the CD4 locus defined as important for regulation by different databases, genomic browsers or described as such in the literature. The regions used are described in **Figure 1.**

**Example 2.2. T-cell specific expression of the CD4 synthetic promoter in a LV background**

LVs expressing eGFP under the regulation of the synthetic CD4 promoter and CEWP-LVs (as control of eGFP expression) were constructed **(Figure 2A).** LVs were generated and used to transduce 293T and BxPC-3 cells as non-CD4-expressing cells, Namalwa (slightly positive for CD4) and Jurkat cells (an immortalized T cell line) and primary human T cells as CD4+++ cells **(Figure 2B, 2C).** These results show that eGFP is only expressed in Jurkat,primary T cells and Namalwa, with almost undetectable levels of expression in 293T and BxPC-3. In primary T cells, the expression of eGFP was similar in CD4 and CD8 subsets in terms of percentage **(Figure 2D, left),** but with an increased intensity in CD4+ population **(Figure 2D, right),** suggesting that the promoter could be used to ensure CAR expression in CAR-T cells. Very low levels of eGFP expression were detected in 293T cells (5.75%), suggesting that the use of this promoter could prevent transgene expression in packaging cells during lentiviral generation. For instance, it could be used to prevent CAR expression on the surface of packaging cells.

**Example 2.3. TCR-like expression of the CD4 polynucleotide**

To analyse if the CD4 promoter-driven LV mimics the TCR expression pattern, the inventors transduced human T cells with CD4-LVs and analysed the kinetics of transgene expression at different times after T cell activation with αCD3/αCD28, as shown in **Figure 3A.** They compared the effects of T cell activation on the percentage of CD3+ cells and eGFP+ cells at different timepoints after stimulation **(Figure 3B).** As previously reported, CD3 expression in T cells decreased soon after stimulation, and basal levels were recovered after 3-7 days. However, such downregulation was not detected in GFP expression, which was driven by the CD4 polynucleotide. Instead, GFP expression levels remained unaltered upon stimulation, suggesting that the use of the CD4 polynucleotide could prevent non-desirable transgene overexpression **(Figure 3B).** The inventors also compared the effects of T cell activation on endogenous CD4 and exogenous CD4-GFP mRNA levels in transduced T cells at different timepoints post stimulation. This revealed that both levels followed similar dynamics, suggesting that expression under the synthetic CD4 promoter follows a CD4-like behaviour

**(Figure 3C).**

In addition, the inventors also determined transgene expression in CD4+ CD3+ and CD8+ CD3+ T cells subsets at different stages of T cells differentiation **(Figure 4).** In CD4+ cells, GFP expression slightly mimicked CD3 expression in CD4+ cells, while it showed a slight increment in CD8+ cells **(Figure 4A).** The highest consistency between the GFP and the CD3 expression profiles was detected in naive/stem memory T cells and central memory T cells, while an increment in GFP compared to CD3 expression was observed in effector T cells. In the context of the generation of CAR-T cells from donors, this analysis evidences the importance of evaluating T cell phenotypes, in order to select less differentiated T cell populations for the generation of CAR-T cells.

## Claims

1. A polynucleotide comprising SEQ ID NO: 7 or having an identity of sequence with SEQ ID NO: 7 of at least 95%.

2. Polynucleotide, according to claim 1, comprising a promoter sequence consisting of SEQ ID NO: 7 or having an identity of sequence with SEQ ID NO: 7 of at least 95%.

3. Polynucleotide, according to any of the claims 1 or 2, comprising a promoter sequence consisting of SEQ ID NO: 7.

4. Polynucleotide, according to any of the claims 1 or 2, consisting of SEQ ID NO: 7.

5. A genetic construct comprising the polynucleotide according to any of the claims 1 to 4.

6. Genetic construct, according to claim 5, comprising the polynucleotide according to any of the claims 1 to 4 as promoter operatively linked to a nucleotide sequence encoding a protein to drive its expression.

7. Genetic construct, according to claim 6, wherein the protein is a chimeric antigen receptor (CAR).

8. Lentiviral vector comprising the polynucleotide of any of the claims 1 to 4, or the genetic construct of any of the claims 5 to 7.

9. T cell or T cell-derived cell comprising the polynucleotide according to any of the claims 1 to 4, a genetic construct according to any of the claims 5 to 7, or any genetic material introduced using the lentiviral vector of claim 8.

10. The polynucleotide according to any of the claims 1 to 4, the genetic construct according to any of the claims 5 to 7, the lentiviral vector according to claim 8, or the T cells or T cell-derived cell according to claim 9, for use in therapy.

11. Use of the polynucleotide according to any of the claims 1 to 4 as a promoter to drive gene expression in T cells or T cell-derived cell.

12. Use, according to claim 11 to drive the expression of a nucleotide sequence encoding a CAR.

13. Use of the genetic construct, according to any of the claims 5 to 7, or the lentiviral vector according to claim 8, to express a nucleotide sequence encoding a CAR in T cells or T cell-derived cells.

14. Method for generating cells that express a transgene in a manner that mimics the expression of the human CD4 gene, which comprises transducing cells with a genetic construct comprising the polynucleotide according to any of the claims 1 to 4 as promoter operatively linked to a nucleotide sequence encoding a protein, or with a genetic construct according to any of the claims 5 to 7, or with the lentiviral vector according to claim 8.

15. Method, according to claim 14, for generating CAR T-cells which comprises transducing T-cells with a genetic construct comprising the polynucleotide according to any of the claims 1 to 4 as promoter operatively linked to a nucleotide sequence encoding a CAR, or with a genetic construct according to any of the claims 5 to 7, or with the lentiviral vector according to claim 8.

**Figure 1**

**A**

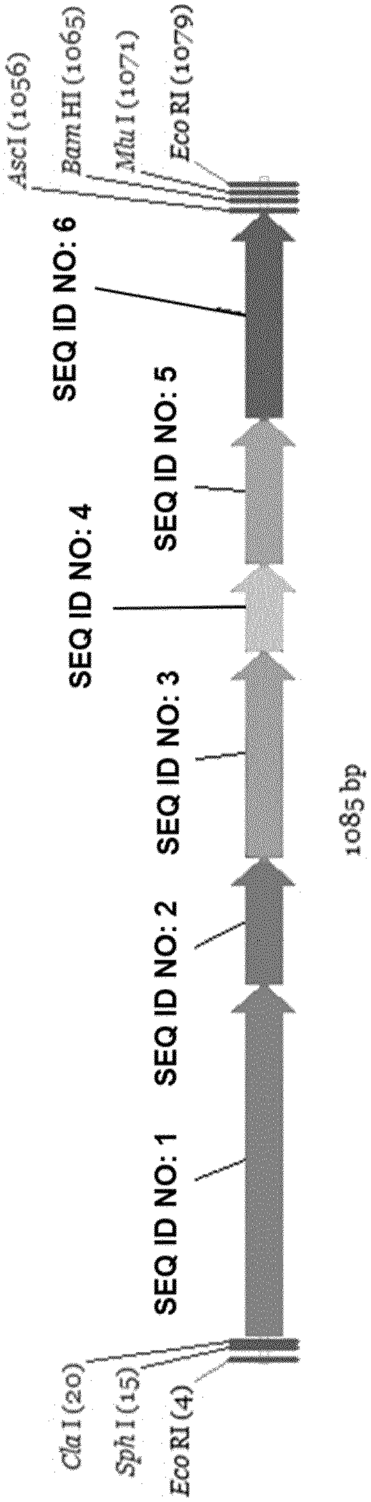
AscI (1056)
Bam HI (1065)
Mlu I (1071)
Eco RI (1079)
SEQ ID NO: 6
SEQ ID NO: 5
SEQ ID NO: 4
SEQ ID NO: 3
SEQ ID NO: 2
SEQ ID NO: 1
1085 bp
Cla I (20)
Sph I (15)
Eco RI (4)

**Figure 1 (cont.)**

**B**

```
     EcoRI    SphI      ClaI
     ~~~~~~ ~~~~~~~~  ~~~~~~~~
   1 TTGAATTCGG CATGCTTATC GATTTTAGGG TTCAGGCCTG AGCCCCTGCT GCTATCCCTC CTTCAAAGGA GGAGATCAAG GAGCTTAGGG TCCCCCGCAC
 101 AGGCCCACCC CAGGGTGGGG TTCTTCCTTT GAAGGGAATT GCTTTGGGGT GGGGTCGGTT CTATCTGCTC CACTCTGTGG CTGACAGTTT CTCCAAGGGG
 201 CTGCAGGTGT CAGCTGTCTG AGCCCGGGCT GAGCTCTGAA ACGTGCCTAC TCAAACTTCC CGTGGGGTAG GGGAGGCCCA GAACCACCCT CTGAGAGTGG
 301 CAAAAAGTGG TCCTGGAGCC AGGGGAAATG TGGATGGGGT AGAGATCTTA TCAAAATTGA GTCACTTGGA CACATCCAGC TGTGAGGGAG GCCTCAACTT
 401 CCTGGAAGTT GGGTTTTATT TTGCTTGGGC AAGTTGGCCA TATGCCCCCA ACTGCCTATT TAAGCCTGAT TCTGCTTAAC TTTTTCCCTT GACTTTGGCA
 501 TTTTCACTTT GACATGTTCC CTGAGAGCCT GGGGGGTGGG GAACCCAGCT CCAGCTGGTG ACGTTTGGGG CCGGCCCAGG CCTAGGGTGT GGAGGAGCCT
 601 TGCCATCGGG CTTCCTGTCT CTCTTCATTT AAGCACGACT CTGCAGAAGG AACAAAGCAC CCTCCCCACT GGGCTCCTGG TTGCAGAGCT CCAAGTCCTC
 701 ACACAGATAC GCCTGTTTGA GAAGCAGCGG GCAAAGACGC AAGCCCAGAG GTAAGGTGGT CAGACTCGGC TTCCTTCCCC GGAGCTGAGA GGGAGGGGAA
 801 CGTGGGGCAG ATGCACAGGA AGAATTTCTG TGCTCTGCCC AGTTGTCTGC TTTAGATAAA TATGTCTGGG GGTTATGGGG AGAAGATAAA AGTGCCTGTG
 901 GGACCACAGA CTCTCGCTGT GGTGGAGCTG GGCCCTCTTA CCCTCCCAAG CCTCGCCCCT CATCCCATCC CTGGGGGCCA GGGGTGAGGG CGGCAGGAAC
                                                                                 MluI
                                                                               ~~~~~~~~
                                                                  AscI      BamHI          EcoRI
                                                                ~~~~~~~~~~ ~~~~~~       ~~~~~~~~
1001 CTCAAGGCTG TGAGAAAGTG CGTGGTGTGT CTTGCCATTT TGGTCTCTTC TCCGGCGCGC CCGGGATCCA CGCGTCTGAA TTCGA
```

**Figure 1 (cont.)**

**C**

| SEQ ID | Sequence ID | Original Location Locus | Size (bp) | Position into the chimeric promoter | Ref | Title |
|---|---|---|---|---|---|---|
| SEQ ID NO: 1 | X70672.1 | 904…1218 | 322 | +25 to +346 | (1)(2) | *Homo sapiens* DNA for CD4 enhancer |
| SEQ ID NO: 2 | NG_027688.2 | 2633…2673 | 116 | +347 to +462 | (3)/ EMBL | *Homo sapiens* CD4 molecule |
| SEQ ID NO: 3 | NG_027688.2 | 4846…5035 | 190 | +463 to +652 | (3) | *Homo sapiens* CD4 molecule |
| SEQ ID NO: 4 | S68043.1 | 365…443 | 79 | +653 to +731 | EPD/ (3) (4) | CD4 {exon 1, promoter} |
| SEQ ID NO: 5 | S68043.1 | 448…558 | 135 | +732 to +866 | EPD/ EMBL | Homo sapiens CD4 molecule |
| SEQ ID NO: 6 | NG_027688.2 | 7650…7835 | 187 | +867 to +1053 | (5) | Homo sapiens CD4 molecule |

Figure 2

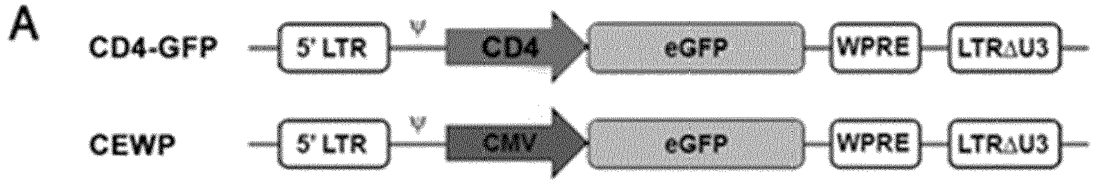
A
CD4-GFP
5' LTR
Ψ
CD4
eGFP
WPRE
LTRΔU3
CEWP
5' LTR
Ψ
CMV
eGFP
WPRE
LTRΔU3

**Figure 2 (cont.)**

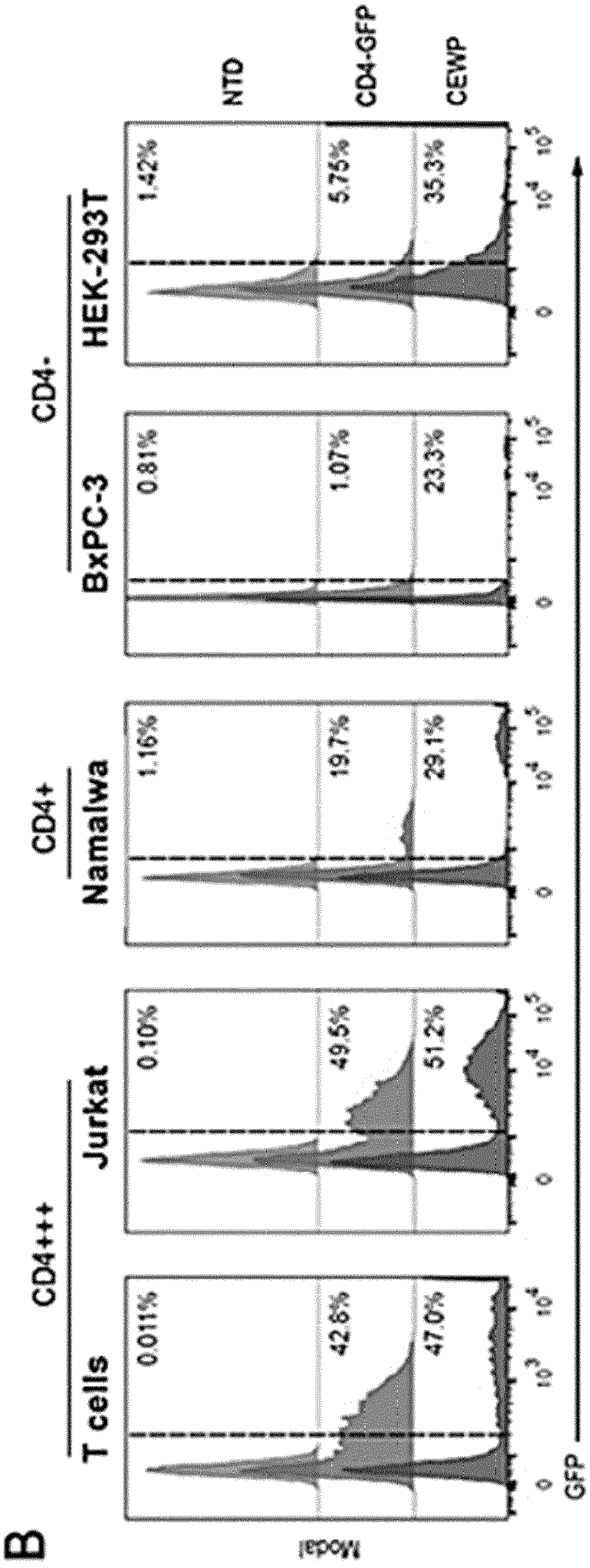

**Figure 2 (cont.)**

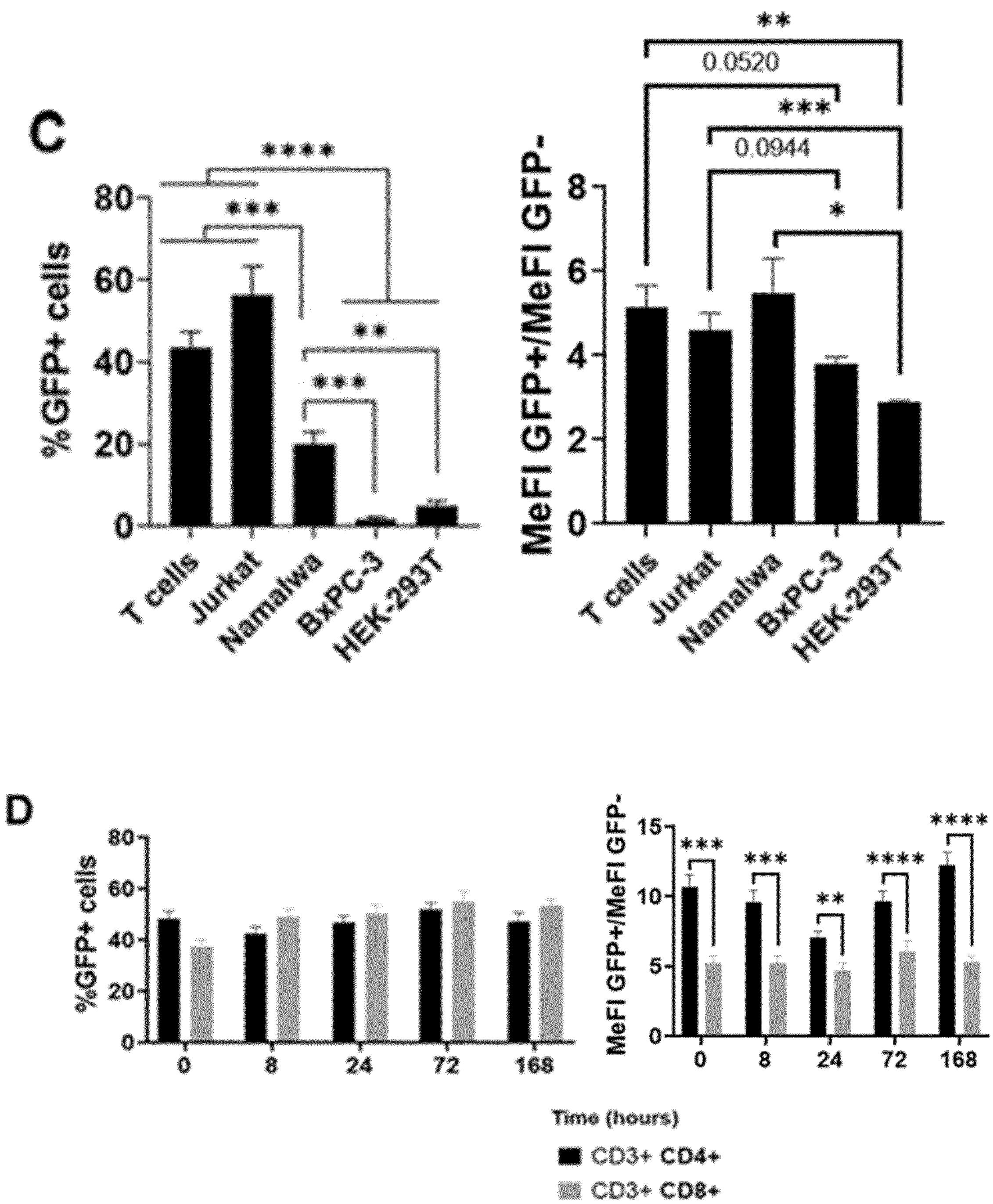

Figure 3

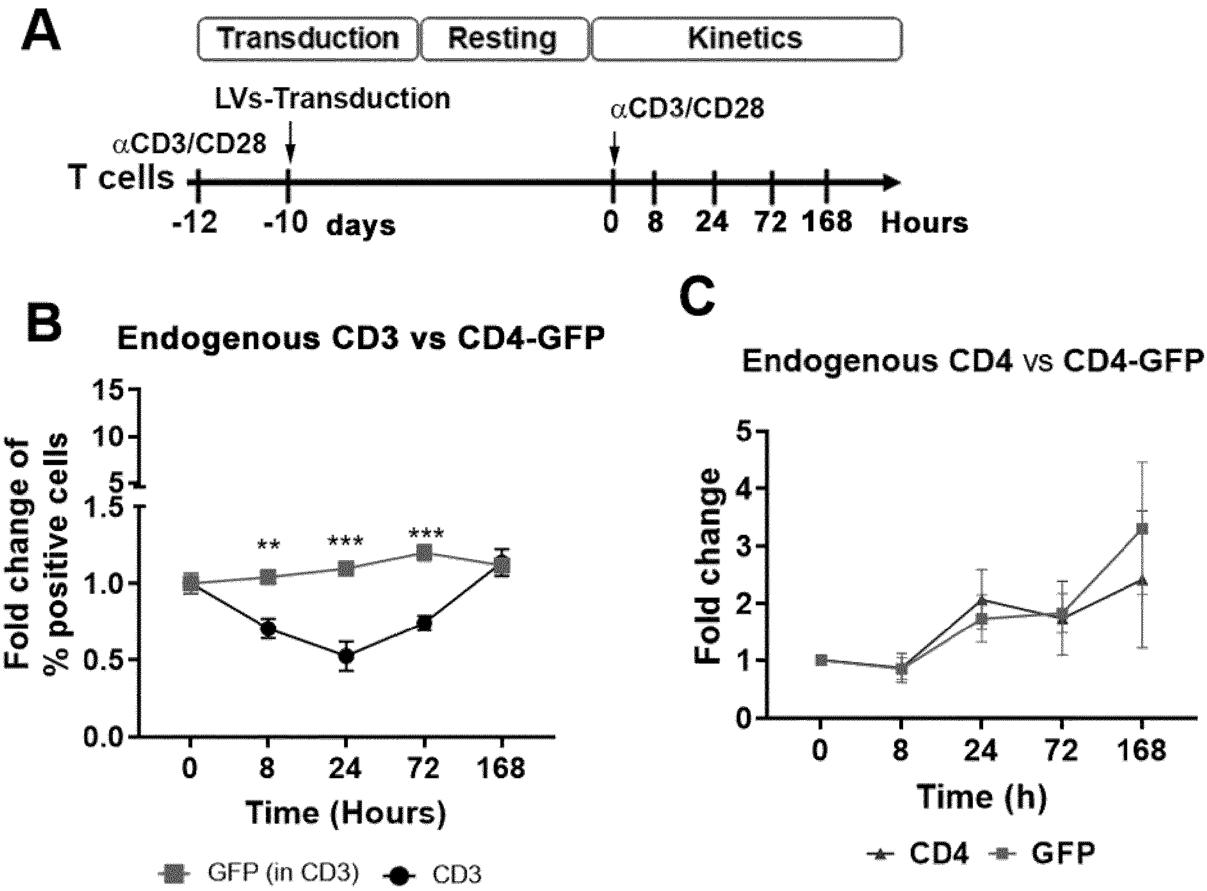
A
Transduction
Resting
Kinetics
LVs-Transduction
αCD3/CD28
αCD3/CD28
T cells
-12
-10
days
0
8
24
72
168
Hours
B
Endogenous CD3 vs CD4-GFP
Fold change of % positive cells
15
10
5
1.5
1.0
0.5
0.0
**
***
***
0
8
24
72
168
Time (Hours)
GFP (in CD3)
CD3
C
Endogenous CD4 vs CD4-GFP
Fold change
5
4
3
2
1
0
0
8
24
72
168
Time (h)
CD4
GFP

Figure 4

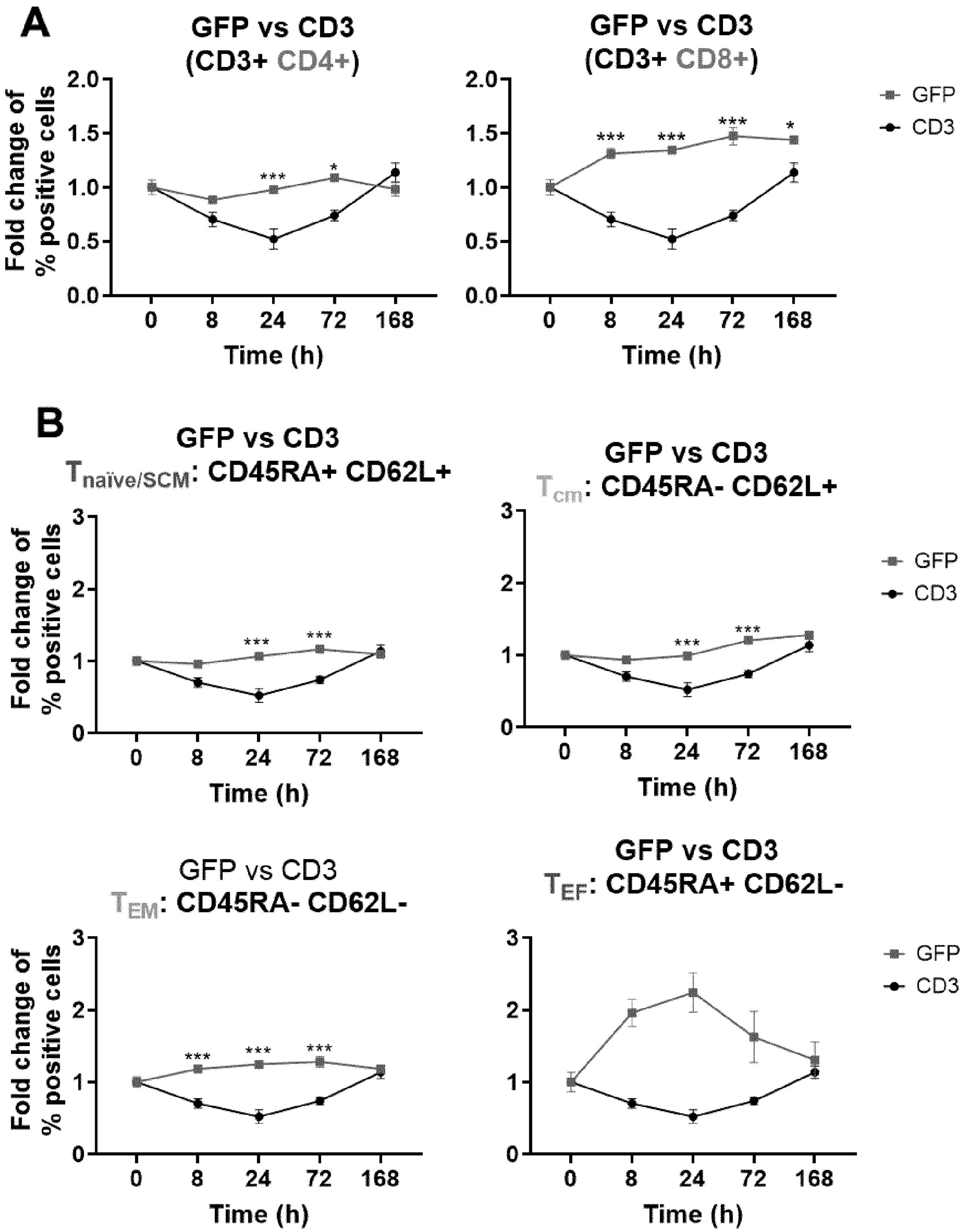
A
GFP vs CD3
(CD3+ CD4+)
GFP vs CD3
(CD3+ CD8+)
Fold change of
% positive cells
Time (h)
GFP
CD3
B
GFP vs CD3
Tnaïve/SCM: CD45RA+ CD62L+
GFP vs CD3
Tcm: CD45RA- CD62L+
GFP vs CD3
TEM: CD45RA- CD62L-
GFP vs CD3
TEF: CD45RA+ CD62L-

Europäisches Patentamt
European Patent Office
Office européen des brevets

# EUROPEAN SEARCH REPORT

Application Number
EP 23 38 2393

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/025800 A1 (AUTOLUS LTD [GB]) 7 February 2019 (2019-02-07) * claims; examples; sequence 1 * ----- | 1-7,9-15 | INV. C07K14/47 A61K39/00 C07K14/725 C12N15/86 |
| X | WO 2020/150534 A2 (BEAM THERAPEUTICS INC [US]) 23 July 2020 (2020-07-23) * claims; examples; sequence 52 * & DATABASE Geneseq [Online] 3 September 2020 (2020-09-03), "Homo sapiens CD4 gene, SEQ 52.", retrieved from EBI accession no. GSN:BIA98305 Database accession no. BIA98305 * sequence * ----- | 1-15 | |

| TECHNICAL FIELDS SEARCHED (IPC) |
|---|
| C07K |
| A61K |
| C12N |

The present search report has been drawn up for all claims

| Place of search | Date of completion of the search | Examiner |
|---|---|---|
| Munich | 6 October 2023 | West, Nuki |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 38 2393

This annex lists the patent family members relating to the patent documents cited in the above-mentioned European search report. The members are as contained in the European Patent Office EDP file on
The European Patent Office is in no way liable for these particulars which are merely given for the purpose of information.

06-10-2023

| Patent document cited in search report | Publication date | Patent family member(s) | Publication date |
|---|---|---|---|
| WO 2019025800 A1 | 07-02-2019 | AU 2018311345 A1 | 27-02-2020 |
| | | CA 3071495 A1 | 07-02-2019 |
| | | CN 111164203 A | 15-05-2020 |
| | | EP 3662055 A1 | 10-06-2020 |
| | | JP 2020530993 A | 05-11-2020 |
| | | JP 2023076572 A | 01-06-2023 |
| | | US 2021130775 A1 | 06-05-2021 |
| | | WO 2019025800 A1 | 07-02-2019 |
| WO 2020150534 A2 | 23-07-2020 | AU 2020208616 A1 | 12-08-2021 |
| | | CA 3126699 A1 | 23-07-2020 |
| | | CN 114072495 A | 18-02-2022 |
| | | EP 3911735 A2 | 24-11-2021 |
| | | JP 2022518463 A | 15-03-2022 |
| | | KR 20210116526 A | 27-09-2021 |
| | | SG 11202107555X A | 30-08-2021 |
| | | US 2022133790 A1 | 05-05-2022 |
| | | WO 2020150534 A2 | 23-07-2020 |

EPO FORM P0459

For more details about this annex : see Official Journal of the European Patent Office, No. 12/82